# EUROPEAN PATENT APPLICATION

(11) **EP 2 573 554 A1**
(43) Date of publication of application: **27.03.2013**
(21) Application number: 11182105.4
(22) Date of filing: 21.09.2011
(51) Int. Cl.: G01N 27/414, G01N 33/543, G01N 33/487

(54) **Apparatus and method for bead detection**

(71) Applicant: NXP B.V., 5656 AG Eindhoven (NL)
(72) Inventor: Frederix, Filip, Redhill, Surrey RH1 1DL (GB); Merz, Matthias, Redhill, Surrey RH1 1DL (GB)
(74) Representative: Burton, Nick

(57) **Abstract**

A bead based assay detection method (and apparatus) is for detecting an analyte, and using a device which comprises a substrate (50), a source region (S), a drain region (D) and a gate region, a channel region (65) between the source and drain regions and a nanopore passing through the substrate in electrical connection with the gate or channel region. A sample under test is drawn through the nanopore, and the sample under test comprises bioreceptors attached to a bead or a bead complex, in proportion to the presence of an analyte to be detected. A count of the beads or bead complexes is made based on modulation of a gate current.

## Description

This invention relates to apparatus and methods for the counting of beads, for example as part of a molecule detection system, for example polymer molecules.

A known an apparatus for counting particles is a Coulter counter, and this is used in many fields from metallurgy to biology.

A Coulter counter can have one or more microchannels that separate two chambers containing electrolyte solutions. As fluid containing particles or cells is drawn through each microchannel, each particle causes a brief change to the electrical resistance of the liquid. The counter detects these changes in electrical resistance.

The particles or cells alter the effective cross-section of the conductive microchannel. If the particles are non-conducting, the electrical resistance across the channel increases, causing the electric current passing across the channel to briefly decrease. By monitoring pulses in electric current, the number of particles for a given volume of fluid can be counted. The size and shape of the electric current signal is related to the size of the particle, enabling a particle size distribution to be measured, and can be correlated to mobility, surface charge, and concentration of the particles. The particle
mobility for example can be derived from the temporal width of a current peak.

The invention relates to a system which can implement a counting function suitable for counting beads in a bead based assay.

According to the invention, there is provided a bead based assay detection method for detecting an analyte, and using a device which comprises a substrate, a source region, a drain region and a gate region, a channel region between the source and drain regions and a nanopore passing through the substrate in electrical connection with the gate or channel region,
wherein the method comprises:
drawing a sample under test through the nanopore, wherein the sample under test comprises bioreceptors attached to a bead or a bead complex, in proportion to the presence of an analyte to be detected; and
performing a count of the beads or bead complexes based on modulation of a gate current.

This method uses driving of a sample containing beads or bead complexes through a nanopore.

The method can comprise:
providing a voltage bias between source and drain regions;
sensing a charge flow between the source and drain regions; and
determining from the sensed charge flow the bead or bead complex count.

The method can comprise determining from the sensed charge flow a bead count in respect of two or more different types of bead, each giving rise to different current flow characteristics. It may also detect the difference between a single bead and a two-bead complex.

The method can comprise:
transporting a sample over a sample preparation area having bioreceptors immobilized on a surface such the analyte binds to the surface;
removing unbound analyte from the surface;
providing a mixture comprising secondary bioreceptors bound to beads, thereby forming sandwich complexes;
washing the surface;
releasing the sandwich complexes, to release secondary bioreceptors bound to beads in a number corresponding to the number of sandwich complexes formed; and
counting the beads.

The sample preparation area can have a plurality of different bioreceptors such that different analytes bind to the surface, and wherein the mixture comprises a plurality of different secondary bioreceptors bound to different kinds of beads, thereby forming a plurality of different sandwich complexes.

This enables different analytes to be detected based on the different properties of the associated beads, which give rise to different current signals. The different kinds of beads can have a different size or material.

In another example, the method comprises:
mixing magnetic beads coated with bioreceptors with the sample, such that the analyte binds to the bioreceptors on the beads.

The beads with bound analyte and the beads without bound analyte can then be counted.

If this counting cannot be performed, the method can further comprise:
transporting the beads with bioreceptors which did not bind the analyte and the beads with bioreceptors and analyte to an area comprising a secondary bioreceptor coated on its surface, thereby forming sandwich complexes;
washing away the beads with bioreceptors which did not have an analyte bound to the surface;
releasing the sandwich complexes, to release bioreceptors bound to beads in a number corresponding to the number of sandwich complexes formed; and
counting the beads.

In another example, the method comprises:
mixing the sample with different bioreceptors bound to beads;
mixing the sample with different secondary bioreceptors bound to beads, such that the different bioreceptors will bind to their respective target and form a sandwich complex in solution;
counting a signal indicating a two-bead sandwich complex pair.

Another method comprises:
mixing the sample with beads coated with different kinds of primary and secondary bioreceptors, such that the primary and secondary antibodies form a sandwich complex with the analyte;
magnetically attracting the beads to a sensor region;
performing a washing step; and
counting a signal indicating a two-bead sandwich complex pair.

Thus, it will be clear that the invention can be applied to many different bead based assay methods.

The invention also provides a bead based assay detector device, comprising:
a substrate;
a source region and a drain region;
a channel region between the source and drain regions;
a gate region connected to the channel region;
a nanopore which passes through the substrate, and which connects fluid chambers on opposite sides of the nanopore, wherein the nanopore passes through the channel region or the gate region;
means for drawing a sample under test through the nanopore; drive means for providing a voltage between source and drain; and
a current sensor for sensing a charge flow between the source and drain regions, wherein the nanopore has a diameter of more than 10nm.

This device uses an enlarged nanopore to enable use in a bead based assay. The nanopore has a diameter matched to (i.e. greater than) the bead dimension, and is more than 10nm. The diameter may be more than 20nm, for example more than 50nm. It may be as large as 1µm.

The nanopore passes through the substrate, and the device may further comprise an insulator layer over the channel region, with the nanopore passing through the insulator layer. The nanopore can pass through a gate electrode.

The invention will now be described in detail with reference to the accompanying drawings, in which:
Figure 1 shows in simplified form a known nanopore detector;
Figures 2a to 2c show first examples of device of US 2010/066348 which can be modified to form a device of the invention;
Figure 3 shows another example of device of the invention;
Figures 4 to 9 show different examples of assay methods of the invention; and
Figures 10a to 10g show an example process flow describing the manufacturing of a nanopore device.

The invention is based on the application of nanopore sensor technology to a bead based assay detection system.

The known use of nanopore sensors will first be discussed, with reference to the main application in that field of DNA nucleotide monomer detection. In particular, the structure of the devices of US2010/066348 which are most suitable for use in accordance with the inventton will first be described, although reference is made to US2010/066348 for further details.

Nanopore sequencing is a favored new technique for DNA sequencing, for example as described in US20050102721 (A1) and US20030104428(A1).

A DNA molecule is 'pulled' through a thin pore with a diameter in the order of a few nanometer by electrophoresis (in most experiments a solid state pore is used, but also transmembrane proteins in a lipid bilayer have been investigated).

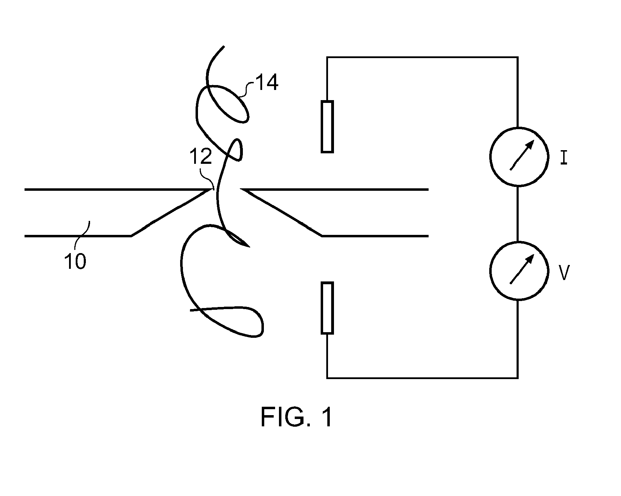

Figure 1 shows the basic operating principle of conventional nanopore sequencing.

A voltage bias across a membrane causes ions to flow through the nanopore 12 between two ion-containing solutions. By drawing polymers of DNA 14 though the nanopore, the number of ions that pass through is reduced, and this is detected as a change in current.

Ideally, the DNA passes the pore one nucleotide at the time, each nucleotide blocking the current through the pore in a characteristic manner. The sequence of current readings directly represents the DNA sequence. In order to be accurate, the pore diameter must be very small (about the size of a nucleotide) so that a single nucleotide can block the current and it must be sufficiently thin to assure that the signal is modulated by a single nucleotide rather than several.

With state-of-the-art nanopore based sequencing techniques, a single nucleotide must be repeated around 50 times followed by an at least equally long sequence of another single nucleotide to produce measurable results. The resolution is clearly too low.

US 2010/066348 of the applicant discloses an arrangement in which a nanopore passes through a channel region, and connects fluid chambers on opposite sides of the nanopore. A voltage bias is driven between the fluid chambers and a voltage is driven between source and drain. A current sensor senses a charge flow between the source and drain regions.

This device uses a nanopore for the confinement of a sample under test (for example nucleotides of a DNA strand) close to a sensor. The size of the sensor can be made similar to the spacing of adjacent nucleotides. In this way, the disadvantages of PCR based techniques are avoided, and single nucleotide resolution can be attained. The device can provide direct contact between the sensor and the sample under test, so that signal disturbance by stray capacitances and resistances are minimized.

The conducting inversion layer of a FET transistor has a thickness in the (sub) nanometer range, which makes it the ideal sensor for sequencing nucleotides which are only separated by 0.34nm in a DNA strand.

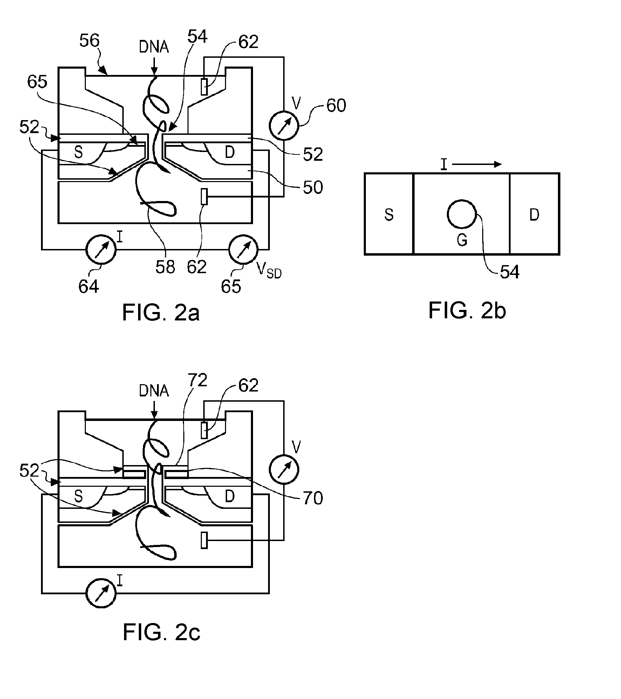

Figure 2a shows the operating principle of a first example of nanopore FET for DNA sequencing, of 2010/066348. A top view is shown in Figure 2b, with source (S) drain (D) and gate (G) indicated, and the position of the nanopore 54.

As shown in Figure 2a, the device corresponds to a field effect transistor having a substrate 50 with source and drain regions S,D, and an overlying gate oxide layer 52. In the following description, the structure is referred to as a transistor, because it has a number of features in common with a transistor, but has a different gate design, as (in some examples) there is no patterned gate electrode. The use of the term "transistor" should be understood accordingly. The structure can, however, be manufactured with minor modification or additional steps to conventional transistor manufacturing processes.

The channel area of the transistor extends between the source and drain, and is provided with a nanopore 54. The nanopore couples upper and lower chambers 56,58 which contain electrolyte. The nanopore has a diameter only slightly larger than the size of the nucleotides (i.e. smaller than 5nm).

The gate voltage of the transistor is provided by the electrolyte voltage of the upper chamber, rather than by a patterned gate electrode. Other than this, the structure of the transistor is conventional, and many different FET designs can be used.

A voltage bias is provided between the fluid chambers by a voltage source 60 associated with electrodes 62, and a current sensor 64 enables sensing of the source-drain current of the transistor. A further voltage source 65 (V_{SD}) also provides a source drain voltage. A further voltage source may be used for controlling the bulk voltage (not shown).

The DNA is moved through the nanopore 54 by applying a voltage between the top and bottom chambers of the cell. This voltage also acts as the gate voltage and sets the working point of the transistor. The nucleotides passing the nanopore modulate the source-drain current if they are in close proximity to the channel.

With this design, the resolution and sensitivity can be so high that individual nucleotides can be detected.

Since the sensitive area of the detector (in vertical direction) is limited to the thickness of the inversion layer, the nanopore can be much longer than in the conventional nanopore devices described above. The sequencing is based on modulation of the source-drain current not by measuring the current through the pore (although synchronous measurement of both currents may be useful for voltage control and feedback).

The relevant operation characteristics of this design which make it suitable for bead detection are:
- The nanopore is located through the substrate of a FET, and the detection is based on modulation of the gate/channel characteristics;
- There is direct modulation of the source-drain current, with no additional conductors and no additional stray capacitances.

Figure 2c shows a second version, in which an additional control gate 70 is provided.

This configuration offers control over the gate voltage independently of the voltage applied between the top and bottom chambers. In order to avoid electrochemical reactions if the voltage between control gate and top electrolyte gets too large, the control gate is insulated by a dielectric 72 from the electrolyte, and this is achieved by not completely removing the passivation or inter-metal dielectric layers on top of the control gate.

Alternatively the insulating dielectric can be deposited (e.g. PVD, CVD) or grown by electrochemical oxidization of the control gate material. An oxide-less configuration incorporating a metal control gate without gate dielectric (similar to a junction FET (JFET)) is also possible. In principle, the insulation layer on top of the control gate can be omitted. Theoretically, the deposition/growth of the gate dielectric 52 in Figure 2a is also not necessary and the gate can be contacted directly by the electrolyte (a thin chemical oxide forms automatically).

A CMOS process for the manufacture of the device is disclosed in US2011/133255.

The device described above is specifically designed for the sequencing of DNA. For this purpose, the pores of the nanopores for DNA sequencing need to be ultra small (a few nm in diameter) to allow single base or molecule sensitivity/specificity. This contributes to the complexity of the processing. Furthermore, the signals for DNA sequencing are rather small since the electrical disturbance is low due to the limited size of the molecules/bases. In addition, by attracting molecules through the nanopore, nonspecific analytes can be attracted through the nanopore. This makes nanopores less suitable for real clinical samples such as blood or urine.

Beads are in general larger (from a few nm up to micrometers). Therefore, the processing challenges are relaxed, which can lead to more standard processing methodologies hence lower cost per sensor.

Thus, the designs of Figures 2a to 2c can be modified to provide a larger pore, which enables simpler processing. The bead detection is then more reliable since the signal greatly exceeds the signal generated by the molecules themselves.

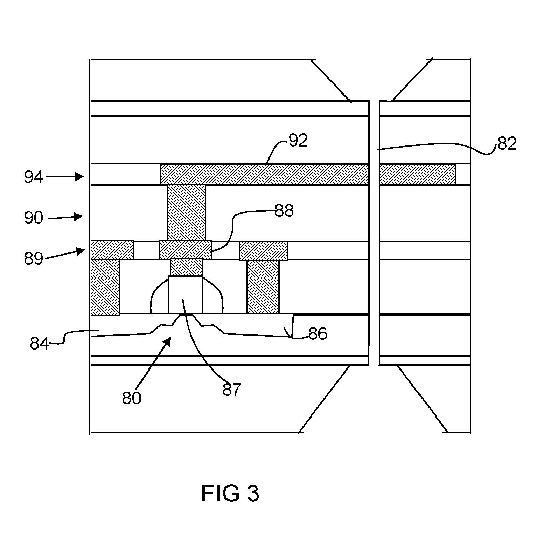

Other (simpler) designs using the same conceptual approach can also be used. Figure 3 shows another example in which the nanopore passes through an extension of the gate electrode, rather than through the channel area. The device comprises a FET80, with the nanopore 82 passing through the substrate to define a channel between opposite sides of the FET stucture.

The FET comprises a source region 84, drain region 86, channel 87 and associated gate electrode 88.

The gate electrode, and source and drain electrodes, are formed in a first metalization layer 89. Using a via in via layer 90, the gate electrode 88 connects to a gate extension 92 in a second metalization layer 94.

The nanopore 82 passes through the gate electrode, and the passage of beads through the gate electrode pore influences the transistor current characteristics by charging the gate electrode and connected gate and modulating the source drain current of the transistor. This design enables simpler processing, in that a simple etch is sufficient. Since the device only has to detect beads (and not the attached molecules themselves) thick metal electrodes can be used, for example with thickness >10nm. For large beads, for example >500nm, the standard intermetal dielectric can be used.

The invention is based generally on the use of a pore-based device, in which the characteristics of a FET type device are modulated by the passage of beads, for Coulter counter type applications.

The diameter of the pore is increased to match the size of the beads. The requirements on sensitivity, noise etc. then become less demanding than for DNA sequencing since the beads are much larger than individual bases resulting in a larger signal.

Since every aperture (pore) has its own detector, large arrays are easy to make and a single fluidic chamber on either side is sufficient. The applied voltage between the fluid chambers only serves to actively drive the beads through the pores by electrophoresis. Alternatively, the beads and liquid could be pumped by a pressure gradient (electrodes are then not necessary in principle; although one electrode may still be needed to define the electrolyte potential, provide ground, reduce noise etc.).

The CMOS compatible integration scheme described in US2011/133255 allows manufacturing of massively parallel arrays with high reliability and at low cost. Moreover, it is possible to integrate electronic circuits for data processing such as amplifiers, filters, A/D converters, threshold detection circuits etc. These can help to reduce the amount of data transferred from the nanopore chip to the external control device, thus further increasing processing speed.

The invention is based on the counting of beads passing through the nanopore and relating them to the concentration of analyte/biomarkers in the solution. Different biological assays can be used and different kinds of beads have to be detected/counted depending on the applied biological assay. Different possible assays are discussed below.

Different bead counting methods can be used, which then relate to a concentration of analyte in the sensor:
1. Different sizes of beads can be used to allow multiplexing or multi-analyte detection. If these different sizes of beads are covered with different bioreceptors to bind different biomarkers, multi-analyte detection can be realized.
2. Different materials of beads can be used to allow multiplexing or multi-analyte detection. If these different material beads are covered with different bioreceptors to bind different biomarkers, multi-analyte detection can be realized. The different materials can be metals, magnetic materials, polymer materials, etc..
3. For beads covered with bioreceptors, the nanopore sensor can measure the difference of a bead covered with an antigen or not covered with the antigen to distinguish between a relevant signal and a non relevant signal.
4. A difference in signal for 1 bead and 2 beads bound to each other via a biological binding event.

These different approaches can be combined.

Different biological assays can be applied in combination with the above possible detection schemes. Various examples of possible assay types are now discussed.

### Example 1

The sample (urine, blood, environmental sample, tissues fluid, etc...) is injected into a system and receives a first sample pre-treatment e.g. filtering, separation, addition of anti-coagulation agents etc.

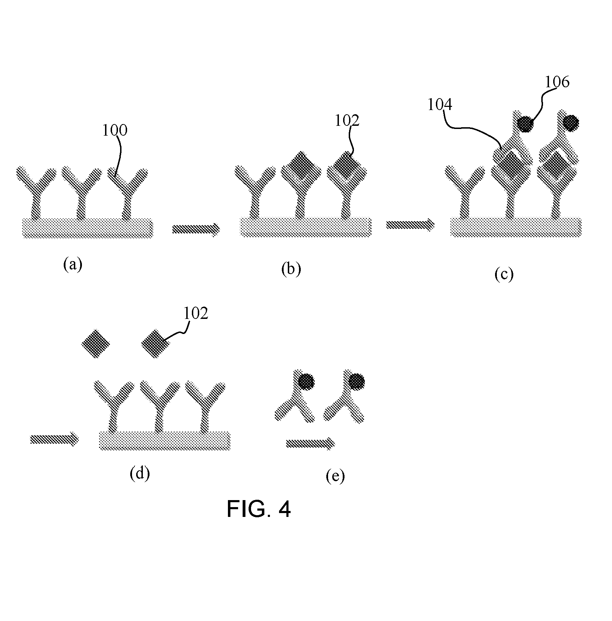

This pre-treated sample is transported over the sample preparation area or the biological assay. This area consists of bioreceptors 100 immobilized on a surface as shown in Figure 4(a). The bioreceptors can be antibodies, DNA, aptamers, antibody fragments etc.

Next, the analyte is transported over this sensor area and the analyte (for example antigen) 102 can bind to the bioreceptor-coated surface as shown in Figure 4(b).

Next, the sensor is washed to remove unbound analyte from the surface and next a secondary bioreceptor (again for example an antibody) 104 coupled to a bead 106 (gold, silver, magnetic, polymer, etc...) is injected over this surface as shown in Figure 4(c). The secondary bioreceptor 104 can bind to the second epitope of the analyte and realize a sandwich assay.

Next, the sensor area is washed, followed by a chemical release of the affinity reaction with adequate buffer solutions. The beads are sent through the CMOS nanopore sensor and the beads are counted. The number of beads is proportional to the amount of analyte 102 bound to the surface hence the concentration of the analyte in the sample.

### Example 2

This is the same as for example 1, but now using DNA/DNA or DNA/RNA or cell/target instead of antibody/antigen interactions.

### Example 3

This is the same as in example 1 or 2 but using different kinds of beads bound to different bioreceptors.

Different kinds of beads can have a different size or materials which give a different signal in the nanopore sensor.

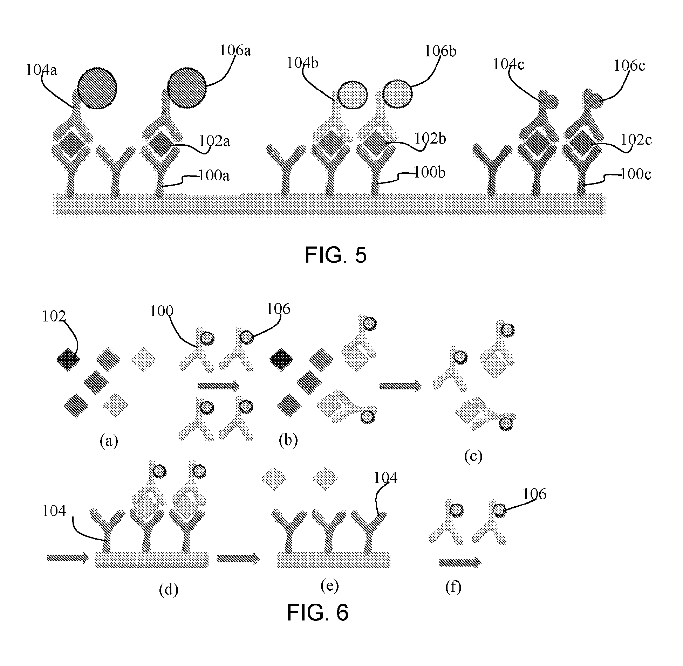

Figure 5 shows this approach. The sensor area has discrete regions covered with particular bioreceptors (such as antibodies) against a particular target. Three different bioreceptors are shown 100a,100b,100c, each associated with a different target 102a,102b,102c. The secondary bioreceptors (such as antibodies) 104a,104b,104c for the particular targets are coupled to different kind of beads 106a,106b,106c. In this way, a multiplex based or multi-analyte sensing method can be performed, since the different beads are involved in a different sandwich assay for a different analyte and their different dielectric properties can be measured with the nanopore sensor.

### Example 4

In this example, the beads are magnetic and covered with the bioreceptors. The bioreceptors bind to the analyte and via magnetic actuation, the beads covered with bioreceptors which can bind the analyte can be transported from the sample to the surface where the biological assay is performed and where the washing steps can take place to increase the specificity. In this case the biological assay flow is shown in Figure 6 and has the following steps:
1. Magnetic beads 106 coated with bioreceptors 100 are mixed with the sample 102 (Figure 6(a)).
2. The analyte can bind to the bioreceptors on the beads (Figure 6(b))
3. The beads with bioreceptors (which did not bind the analyte) and the beads with bioreceptors and analyte (shown in Figure 6(c)) are transported to an area where a secondary bioreceptor 104 is coated on the surface (Figure 6(d)).
4. A sandwich assay is realized (Figure 6(d)) and the beads with bioreceptors which did not have an analyte (antigen) will not be bound to the surface
5. Next, the affinity reactions are broken by an adequate buffer rinse (Figure 6(e)) and the beads are transported to the nanopore to count and relate it to the original nanoparticle concentration (Figure 6(f))

### Example 5

This example is the same as example 4, but if the nanopore sensor can measure the difference between an analyte (such as an antigen) bound to the beads and beads which do not have an analyte, then the process flow can stop at Figure 6(c) and the beads can be immediately transported to the nanopore sensor.

### Example 6

This example is the same as example 5 or 6, but now using DNA/DNA or DNA/RNA or cell/target or DNA/protein.

### Example 7

This example is the same as in example 5 or 6 but in combination with different kinds of beads as shown in Figure 5.

### Example 8

This is an example of a solution based assays. This has the advantage that it does not need an area where a biological assay should take place. This results in easier microfluidic concepts, but there is no possibility to have washing steps. These washing steps are used to increase the specificity, to see the difference between bioreceptors which did bind their target and bioreceptors which did not bind their target.

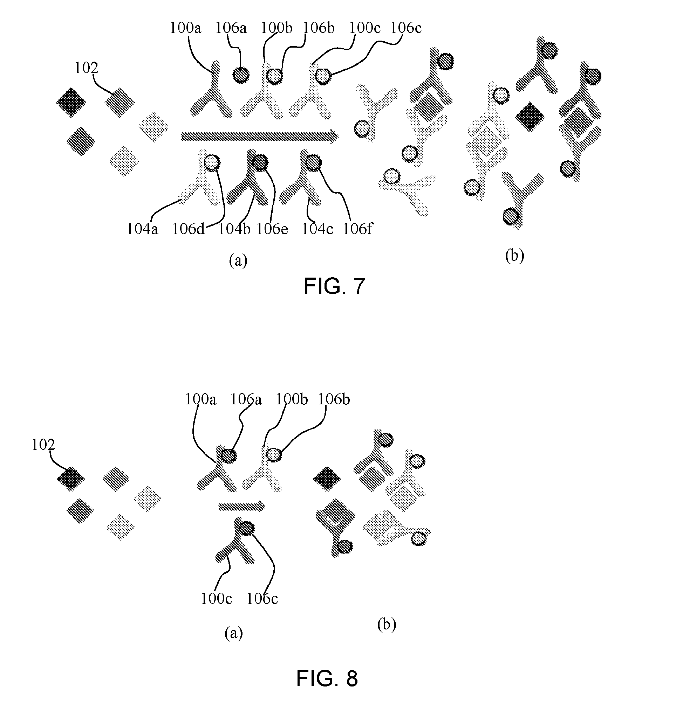

Therefore other biological methodologies have to be used. A methodology to overcome this disadvantage is to realize a sandwich assay in solution and transport the sandwich complex through the nanopore sensor. This approach is shown in Figure 7.

This method has the following steps:
1. The sample is mixed with different bioreceptors (such as antibodies) 100a-100c and 104a-104c bound to different kind of beads 106a-106f as shown in Figure 7(a). The different bioreceptors target a different analyte. Furthermore, the sample is mixed with primary and secondary bioreceptors 100a-100c and 1 04a-1 04c bound to beads 1 06d-1 06f.
2. The different bioreceptors will bind to their respective target and form a sandwich assay in solution as shown in Figure 7(b).
3. The solution containing the sandwich complexes and containing the bioreceptors bound to the beads (but not in a sandwich complex) can be transported to the nanopore sensor.
4. The sensor records the signal of two beads in the sandwich assay. The signal of two beads is representative for one analyte. The signal for one bead should not be counted into the signal.

### Example 9

This example is another solution based assay as for example 8, but with beads which are magnetic. If these beads are used in the sandwich complexes, they can be drawn through the nanopore sensor to increase the speed of the total assay.

### Example 10

This example is the same as example 9 but with different beads: If different kinds of beads are used, multi-analyte detection is possible since different beads can give a different signal.

### Example 11

This example can be based on examples 8 to 10, but using DNA/DNA or DNA/RNA or cell/target or DNA.

### Example 12

This example is based on the initial steps of examples 8 to 12, but if the nanopore sensor can measure the difference between an analyte bound to the beads and beads which do not have an analyte then the process flows in examples 8-12 can be shortened. Figure 8 is an example.

The different steps are:
1. The sample is mixed with bioreceptors 100a-100c bound to different beads 106a-106c as shown in Figure 8(a), to give the arrangement shown in Figure 8(b).
2. The sample is sent to the nanopore sensor to measure the difference between beads bound to analyte and beads which are not bound to an analyte.

### Example 13

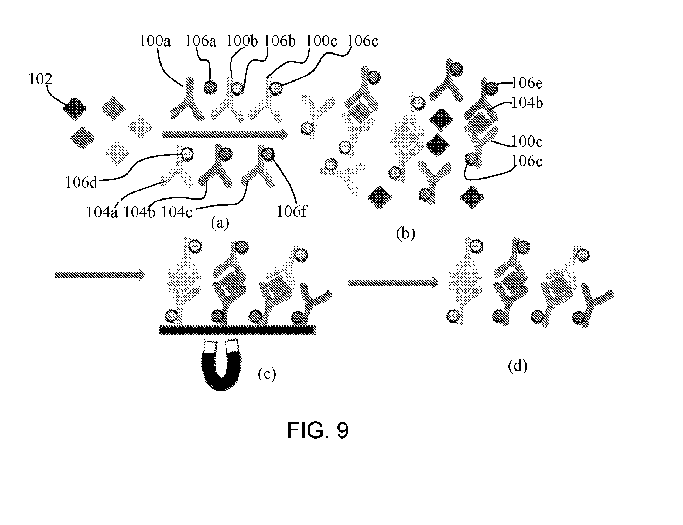

If the analyte is contained in a complex sample, it would be useful to separate the majority of the non interesting proteins/cells from the matrix. This can be realized by filtering techniques but also by magnetic attraction of the analyte of interest while washing the unwanted material out of the matrix. Using magnetic beads in combination with nanopore sensing can therefore be a full solution for measuring in a clinical sample. The biological assay flow is shown in Figure 9.

The different steps are
1. The sample is mixed with beads 106a-106f coated with different kinds of bioreceptors 100a-100c and 104a-104c (Figure 9(a)) including primary and secondary bioreceptors..
2. The primary and secondary bioreceptors will form a sandwich complex with the analyte (Figure 9(a)).
3. This sandwich complex is magnetically attracted to a certain region onto the sensor (Figure 9(c)) followed by a washing step to remove unwanted species from the matrix.
4. Next, the sandwich complexes are transported through the nanopore sensor where a difference is measured between the sandwich complex (with two beads) and the beads coated with a single bioreceptor (one bead).

### Example 14

This example is the same as example 12 (Figure 8) but including the magnetic washing from example 13 (Figure 9).

These examples provide an indication of the range of assays that can be used with the nanopore structure when used for bead detection.

Most steps for the implementation/manufacturing of the sensor device are straightforward. Except for the pore creation step, they are essentially identical to a state-of-the-art CMOS process (e.g. C065).

In the known implementation for nanopore sensing, additional processing steps are required to provide a reduced thickness of the stack through which the pore passes and the formation of the nanopore itself. These thinning steps may not be required in the bead assay application.

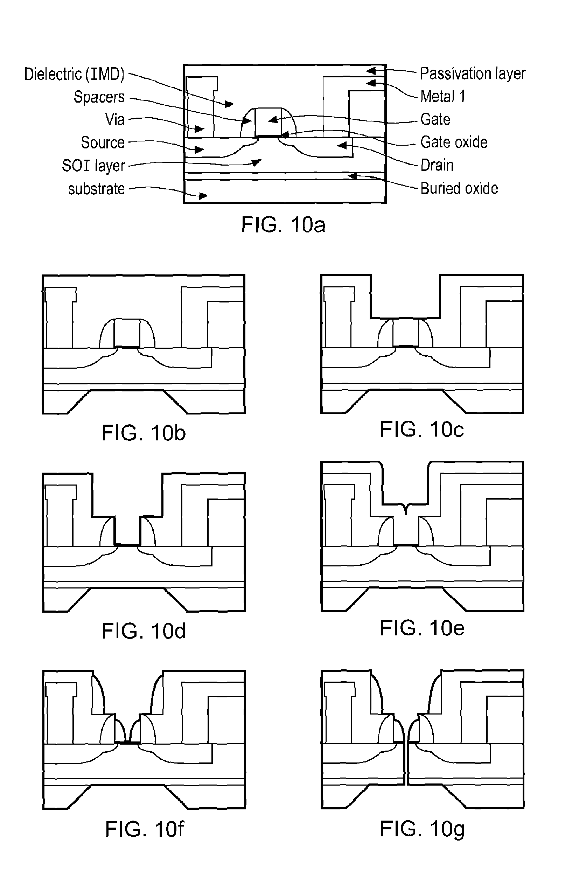

For completeness, the (known) steps for manufacturing the device of Figure 2a to 2c are described below with reference to Figure 10 (not drawn to scale). Note that only the essential steps are shown, standard procedures such as resist deposition lithography etc. are omitted for clarity.

The starting point is a device/wafer that underwent a full 'conventional' process flow including passivation layer deposition and bondpad patterning; such a device is shown in Figure 10a with all relevant components indicated (several components e.g. the inter metal dielectric (IMD) may consist of several layers of different materials; not shown in detail). The backside of the wafer is locally thinned/etched beneath the channel region such that only a thin layer of Si substrate (e.g. less than 100nm thickness) remains underneath the gate oxide. The thinner the stack i.e. the shorter the pore, the easier it is to pull the DNA through. The resulting taper is shown e.g. in Figure 2a. This step can easily be done if the device is processed on silicon-on-insulator wafers. In this case the buried oxide layer can serve as etch stop during the etch of the bulk wafer see Figure 10b.

The passivation layer in the gate region is then locally removed from the top side (by etching) (Figure 10c) followed by etching away the gate stack (Figure 10d). If needed the gate oxide can be removed and another gate dielectric can be deposited. The final steps comprise etching of the pore e.g. according to the scheme presented in Figure 10e to 10g and attachment of the electrolyte chambers.

Etching of small pores with diameters of less than 10nm and very high aspect ratios above ten is a major challenge in the manufacturing of these devices. While such small structures can be written with e-beam lithography they are difficult to implement with standard CMOS process technology.

Figures 10e to 10g show how small pores can be obtained with conventional lithography. First, a small opening/cavity (e.g. quadratic or circular) is etched into a hard-mask e.g. oxide or nitride layer. In Figure 10d this opening is defined by the hole remaining after the etch of the gate stack. With state-of-the-art optical lithography equipment feature sizes in the range of 50nm can be reached for such a hole. The aperture of this hole is further reduced by spacers shown in Figure 10f, a standard process module in the production of MOS transistors. The process involves the following steps: uniform deposition of an e.g. oxide or nitride layer across the hole and the surrounding areas (Figure 10e), followed by anisotropic etching until a small aperture opens at the bottom (Figure 10f). This aperture serves as a mask for the subsequent etching of the pore with the desired diameter (greater than 10nm) (Figure 10f), In the selection of materials, care must be taken that enough etch selectivity is provided between the mask and the materials to be etched.

Alternatively, the pore can be 'drilled' with a focused ion beam or prepared with methods known from the manufacturing of conventional nanopores (e.g. ion/electron-beam sculpturing). If silicon on insulator wafers are used, the final thickness of the stack through which the pore passes (i.e. the length of the pore) is given by the thickness of the buried oxide (minus the oxide removed during the etching of the bulk substrate), the thickness of the silicon on insulator layer and the gate oxide thickness. Preferentially the overall thickness is less than 200nm.

The blank silicon surfaces in the pore rapidly oxidize in the aqueous environment of the electrolyte forming a thin 'gate' oxide along the pore walls. This is shown in Figure 2a, with reference 52 showing the different oxide layers.

If needed, the thickness of these insulation layers can be increased by local oxidation e.g. by heating with a laser beam or by strong oxidation solutions. Moreover additional dielectrics (oxide, nitride) can be deposited on the top and bottom on either side of the pore. Electrochemical processes or ALD (atomic layer deposition) may also be used.

The device of Figure 3 can be manufactured more simply. Essentially,. The design comprises a standard device with a hole/pore etched through the entire chip, with some thinning of wafer substrate at backside. A silicon on insulator structure can again be used, but this is not essential since pore has a large diameter and can be etched through thick layers, without issues arising from high aspect ratios.

The detailed processes required will be apparent to those skilled in the art, and outline descriptions of the processes have been given. Some embodiments may not need all steps of a standard CMOS flow e.g. with the heterostructure device the extension implant is not needed. There are many other ways to produce the devices which will be routine to those skilled in the art.

The description above is limited to features and processes for MOSFETs. However, the dielectric layer of these devices with its large number of surface and interface charges and traps cause considerable noise which may prevent the detection of the weak signals from the nucleotides. In order to minimize this noise, JFETs can be used. The gate is then directly contacted by a metal thus avoiding any oxides and their specific problems. Again the metal can be contacted and controlled by the electrolyte or a separate (gate) contact.

Although only single pores have been discussed above, CMOS process technology allows the manufacturing of large arrays of nanopore FETs enabling massive parallel sequencing at high speed. Moreover signal conditioning circuits such as amplifiers, filters and ADCs (analog to digital converter) may be implemented on the same chip/die in close proximity to the sensor device. This prevents signal loss e.g. in the otherwise long connection lines to external amplifiers and can be essential for the detection of the small signals with a sufficient signal-to-noise ratio.

The device of the invention can have fluid chambers which are part of a micro fluidic system transporting analyte to the detector device. Signal conditioning circuits can also be provided, such as amplifiers, filters and ADCs (analog to digital converters) on the same chip. These signal conditioning circuits can be used to improve the Signal-to-Noise ratio of the detected signals. A device can incorporate several of the individual detector devices on a single chip/die.

Various modifications will be apparent to those skilled in the art.

## Claims

1. A bead based assay detection method for detecting an analyte, and using a device which comprises a substrate (50), a source region (S), a drain region (D) and a gate region, a channel region (65) between the source and drain regions and a nanopore passing through the substrate in electrical connection with the gate or channel region,
wherein the method comprises:
drawing a sample under test through the nanopore, wherein the sample under test comprises bioreceptors attached to a bead or a bead complex, in proportion to the presence of an analyte to be detected; and
performing a count of the beads or bead complexes based on modulation of a gate current.

2. A method as claimed in claim 1, comprising:
providing a voltage bias between source and drain regions;
sensing a charge flow between the source and drain regions; and
determining from the sensed charge flow the bead or bead complex count.

3. A method as claimed in claim 1 or 2, comprising determining a bead count in respect of two or more different types of bead.

4. A method as claimed in claim 1, comprising:
transporting a sample over a sample preparation area having bioreceptors (100) immobilized on a surface such the analyte (102) binds to the surface;
removing unbound analyte (102) from the surface;
providing a mixture comprising secondary bioreceptors (104) bound to beads (106), thereby forming sandwich complexes;
washing the surface;
releasing the sandwich complexes, to release secondary bioreceptors (104) bound to beads (106) in a number corresponding to the number of sandwich complexes formed; and
counting the beads.

5. A method as claimed in claim 4, wherein the sample preparation area has a plurality of different bioreceptors (100a-100c) such that different analytes (1 02a-1 02c) bind to the surface, and wherein the mixture comprises a plurality of different secondary bioreceptors (104a-104c) bound to different kinds of beads (106), thereby forming a plurality of different sandwich complexes.

6. A method as claimed in claim 5, wherein the different kinds of beads have a different size or material.

7. A method as claimed in claim 1, comprising:
mixing magnetic beads (106) coated with bioreceptors (100) with the sample (102), such that the analyte binds to the bioreceptors on the beads.

8. A method as claimed in claim 7, further comprising:
counting the beads with bound analyte and beads without bound analyte.

9. A method as claimed in claim 5, further comprising:
transporting the beads with bioreceptors which did not bind the analyte and the beads with bioreceptors and analyte to an area comprising a secondary bioreceptor (104) coated on its surface, thereby forming sandwich complexes;
washing away the beads with bioreceptors which did not have an analyte bound to the surface;
releasing the sandwich complexes, to release bioreceptors (100) bound to beads (106) in a number corresponding to the number of sandwich complexes formed; and
counting the beads.

10. A method as claimed in claim 1, comprising:
mixing the sample with different bioreceptors (100a-100c) bound to beads (106a-106c);
mixing the sample with different secondary bioreceptors (104a-104c) bound to beads (106d-106f), such that the different bioreceptors will bind to their respective target and form a sandwich complex in solution;
counting a signal indicating a two-bead sandwich complex pair.

11. A method as claimed in claim 10, wherein the beads are magnetic.

12. A method as claimed in claim 10, wherein different kinds of bead are used for each different sandwich complex type.

13. A method as claimed in claim 1, comprising:
mixing the sample with beads (1 06a-1 06f) coated with different kinds of primary and secondary bioreceptors (100a-100c, 104a-104c), such that the primary and secondary antibodies form a sandwich complex with the analyte;
magnetically attracting the beads to a sensor region;
performing a washing step; and
counting a signal indicating a two-bead sandwich complex pair.

14. A bead based assay detector device, comprising:
a substrate (50);
a source region (S) and a drain region (D);
a channel region (65) between the source and drain regions;
a gate region connected to the channel region;
a nanopore (54) which passes through the substrate, and which connects fluid chambers (56,58) on opposite sides of the nanopore, wherein the nanopore passes through the channel region or the gate region;
means (60) for drawing a sample under test through the nanopore;
drive means for providing a voltage between source and drain V_{SD}; and
a current sensor (64) for sensing a charge flow between the source and drain regions, wherein the nanopore has a diameter of more than 10nm.

15. A device as claimed in claim 14, wherein the nanopore passes through a gate electrode.
